# EUROPEAN PATENT SPECIFICATION

(11) **EP 2 211 857 B1**
(45) Date of publication and mention of the grant of the patent: **12.09.2012**
(21) Application number: 08805179.2
(22) Date of filing: 09.10.2008
(51) Int. Cl.: A61K 31/4425, A61K 35/00, A61P 39/06

(54) **USE OF QUATERNARY PYRIDINIUM SALTS FOR RADIOPROTECTION**
VERWENDUNG VON QUARTÄREN PYRIDINIUMSALZEN ALS STRAHLENSCHUTZ
UTILISATION DE SELS DE PYRIDINIUM QUATERNAIRE POUR LA RADIOPROTECTION

(30) Priority: 12.10.2007 EP 07465007; 12.10.2007 US 998856 P; 09.01.2008 EP 08465001; 09.01.2008 US 10487 P
(43) Date of publication of application: 04.08.2010
(73) Proprietor: Politechnika Lodzka, 90-924 Lodz (PL); Uniwersytet Jagiellonski, 31007 Krakow (PL)
(72) Inventor: GEBICKI, Jerzy, PL-94-217 Lódz (PL); MARCINEK, Andrzej, PL-91-496 Lódz (PL); CHLOPICKI, Stefan, PL-30-047 Kraków (PL); JANIAK, Marek, PL-03-982 Warszawa (PL)
(74) Representative: Sitkowska, Jadwiga
(86) International application number: PCT/EP2008/063534
(87) International publication number: WO 2009/047290

(56) References cited:
- WO-A-2005/067927
- WO-A-2007/074406
- WO-A-2007/103540
- WO-A-2008/104920
- US-A1- 2006 160 805
- ALLEN J B ET AL: "Irradiation decreases vascular prostacyclin formation with no concomitant effect on platelet thromboxane production." LANCET 28 NOV 1981, vol. 2, no. 8257, 28 November 1981 (1981-11-28), pages 1193-1196, XP008101126 ISSN: 0140-6736
- MILAS ET AL: "Cyclooxygenase-2 (COX-2) enzyme inhibitors as potential enhancers of tumor radioresponse" SEMINARS IN RADIATION ONCOLOGY, SAUNDERS, PHILADELPHIA, PA, US, vol. 11, no. 4, 1 October 2001 (2001-10-01), pages 290-299, XP005492681 ISSN: 1053-4296

## Description

### The field of the invention

The present invention relates generally to the field of radioprotection, including radioprotection in a radiotherapy (radiation therapy). In particular it relates to the treatment or prevention of a cell, tissue or organ damage in a living subject caused by the exposure to the ionizing radiation of any type. The invention comprises administration of effective amounts of certain quaternary pyridinium salts to a subject, including human.

### The state of the art

Ionizing radiation, i.e. the radiation with the energy high enough to have the potential of the ionization of molecules in a living body, may cause serious damages and injuries to the cells and tissue of living beings. Ionizing radiation damages tissue by direct ionization, which disrupts molecules directly and also by producing highly reactive free radicals, which attack nearby cells. The net effect is that biological molecules suffer local disruption; this may exceed the body's capacity to repair the damage and may also cause mutations in cells currently undergoing replication. In consequence, a dysfunction of many important organs, and even multiple organ failure is observed, which can eventually lead to a death (radiation-induced lethality). Damaging and harmful effects of the radiation can be observed both in the case of acute high dose exposure or in the case of chronic exposure to lower doses. These include so called radiation sickness caused by chronic exposure to the radiation emitting environment, and radiation sickness (poisoning), caused by acute exposure to the internal or external action of a radioactive material or a source of radiation. Chronic exposure to low doses has mutagenic activity and brings a risk of developing cancer. Harmful effects of the radiation can be also due to the exposure of a patient or a medical staff to the radiation during routine radiodiagnostic procedures or a radiotherapy, like radiotherapy of a cancer, where radiation which destroys cancer cells can at the same time damage healthy, normal cells.

Some agents have been approved for eliminating radioactive substances from the body, among them radiogardase (prussian blue), pentetate calcium trisodium (Ca-DTPA) and pentetate zinc trisodium (Zn-DTPA).

Chemicals capable of protecting the cells and tissue against harmful effects of the radiation, called radioprotectors, are used and tested in clinical trials. Among them aminothiol compounds, like mercaptamine, glutation, amifostine and their phosphorylated pro-drugs, have been developed. Aminothiole protectants exert their action due to their free-radicals scavenging and antioxidant ability and to provide effective protection must be given prior to the exposure.

The disadvantage and the main limitation of the classical aminothiol-based radioprotectors is their high toxicity, especially at concentrations required for radioprotection, low efficiency when used after the radiation exposure, lack of protection against radiation-induced lethality/mortality, and generally low degree of protection. In the case of cancer radiotherapy a potential disadvantage is a risk of compromising cancer cells kill when radioprotector reaches tumor cells.

In the recent years the interest in biological treatments which could be administered after the exposure could have been observed. This included the use of agents which could increase survival after accidental radiation injuries: anti-apoptotic proteins, cell growth factors, G-CSF and GM-CSF (filgrastim). These drugs stimulate the growth of white blood cells and can help repair bone marrow damage. They can be also used in patients receiving radiation therapy.

Due to extensive use and presence of ionizing radiation and/or radiation sources in many fields of human activity, such as medicine, nuclear power plants, industry, as well as the threat of contamination caused by nuclear/terrorist attacks the need still exists for radioprotectors based on simple chemical molecules, which could be effective especially in preventing radiation-induced lethality while being non-toxic and safe at concentrations required for effective protection. It is important as well that potential radioprotection candidate is relatively cheap and easy to manufacture.

The protection against radiation caused damage discovered by the present inventors employs simple and cheap small molecules - certain quaternary pyridinium salts.

Certain therapeutic uses of quaternary pyridinium salts are known.

In WO00/40559 therapeutic and cosmetic uses of 1,3-disubstituted pyridinium salts, including I-methylnicotinamide (MNA) salts were disclosed. It was reported that said derivatives have the utility in topical treatment of skin diseases, in particular crural ulceration, acne, psoriasis, atopic dermatitis, vitiligo, as well as bums and scalds and in wound healing. Said derivatives have also the activity of promoting hair re-growth, therefore they are useful in the treatment of hair loss of different origin.

Effects of 1-methylnicotinamide chloride (MNA) in some skin diseases were described by Gçbicki J, Sysa-Jçdrzejowska A, Adamus J, Wozniacka A, Rybak M, Zielonka J. 1-.Methylnicotinamide: a potent anti-inflammatory agent of vitamin origin. Pol. J. Pharmacol. 2003;55:109-112. It has been proposed that MNA displays anti-inflammatory action.

In WO2005/067927 the use of 1-methylnicotinamide and 1-methyl-3-acetylpyridinium salts as vasoprotective agents in the treatment of conditions and diseases associated with endothelial dysfunction was disclosed.

In Pharmacological Reports, 2007, 59, 216-223, studies of cytotoxic activity of selected pyridinium salts: 1-methylnicotinamide chloride, 1-methyl-3-acetylpyridinium chloride and 1-methyl-3-nitropyridinium chloride against murine leukemia L1210 were reported by the present inventors. It was found that the cytotoxicity in cultured leukemia cells varied strongly depending on the compound used and that 3-nitro-1-methylpyridinium chloride showed the highest cytotoxicity, while cytotoxicities of 1-methylnicotinamide chloride and 1-methyl-3-acetylpyridinium chloride were several thousands times lower, thus being in fact negligible. Furthermore, the activity of 1-methylnicotinamide chloride was observed only at very high concentration.

1-Methyl-3-acetylpyridinium salt (MAP⁺) was described in a publication Takashi Sakurai, Haruo Hosoya. Charge transfer complexes of nicotinamide-adenine dinucleotide analogs and flavine mononucleotide. Biochim. Biophys. Acta 1966;112(3):359-468.

US 2006/0160805 describes tetrathiotungstate bis(quaternary ammonium) compounds as copper chelators which can be used as angiogenesis inhibitors inter alia for the treatment of cancer. It is also suggested that the compounds can be used in combination with other active therapeutics, such as e.g. radiation therapy. Bis(1,4-dimethylpyridinium) tetrathiotungstate is one of the exemplified compounds; the combined cancer treatment using this compound and radiotherapy is not explicitly disclosed.

### BRIEF DESCRIPTION OF THE DRAWINGS

The following drawings form part of the present specification and are included to further illustrate and demonstrate certain aspects of the present invention.

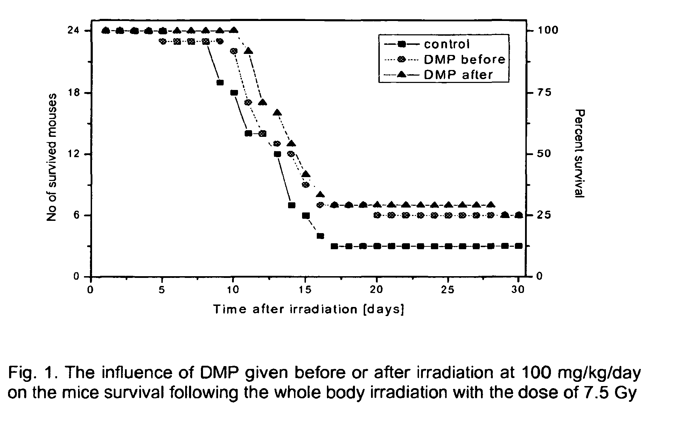

Fig. 1 shows the effect of the administration of 1,4-dimethylpyridinium chloride (DMP) at 100 mg/kg/day given before or after irradiation on the survival rate of mice after whole body irradiation with the dose of 7.5 Gy in comparison with the untreated group of mice.

### Summary of the Invention

There is provided a quaternary pyridinium salt of formula (I) 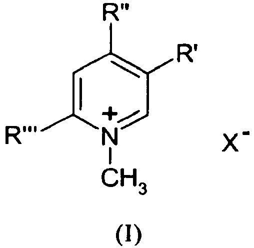 wherein R' is H, R"' is H or CH₃, and X⁻ is a pharmaceutically acceptable counterion, for use in the treatment or prevention of a cell, tissue or organ injury in a living subject due to the exposure to ionizing radiation.

Said treatment or prevention comprises administration to said subject of a quaternary pyridinium salt of formula (I) 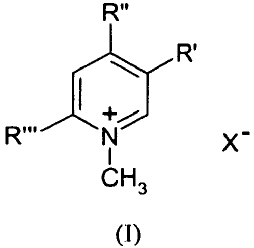 wherein R' is H, and R" is H or CH₃, R"' is H or CH₃, and X⁻ is a pharmaceutically acceptable counterion, in an amount sufficient to inhibit radiation-induced damage.

It has been unexpectedly found that the compounds of the above formula (I), while being devoid of any radical scavenging and antioxidant activity, possess the superior potential to protect living subjects against harmful effects of the ionizing radiation, including radiation sickness, radiation-induced lethality as well as harmful effects of radiotherapy to normal/healthy cells. Their superior effect can be observed after oral administration, although is much quicker after administration via parenteral route. The advantage which is very important especially in emergency cases is a quick onset of action. They show high efficiency when administered following the exposure to the ionizing radiation. The compounds of formula (I) are quickly redistributed in the body, do not accumulate during chronic/prolonged administration, and are devoid of side effects. Furthermore, they show efficiency in the case of the exposure to any type of ionizing radiation and in the case of both irradiation of a whole body of the living subject and particular organs of the body.

### Detailed disclosure of the invention

There is provided a quaternary pyridinium salt of formula (I) 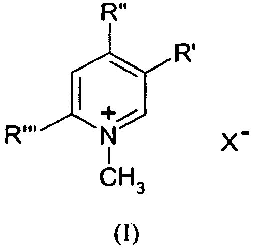 wherein R' is H, R" is H or CH₃, R''' is H or CH₃, and X⁻ is a pharmaceutically acceptable counterion, for use in the treatment or prevention of a cell, tissue or organ injury in a living subject due to the exposure to ionizing radiation.

As indicated above, in the compounds of formula (I) X⁻ can be any pharmaceutically acceptable and physiologically suitable counter-anion. Said counterion is of a non-complex type, i.e. does not contain complexed metal cations. The quaternary pyridinium salts used in the present invention can thus be derived from any physiologically suitable acceptable organic or inorganic acids. Suitable inorganic acid salts include, for example, chloride, bromide, iodide and carbonate. Suitable organic acid salts include for example mono-, di-and tri-carboxylic acid salts such as acetate, benzoate, salicylate, glycolate, lactate, maleate and citrate.

The compounds of the invention are useful in any types of the exposure, whether acute or chronic, to any type of ionizing radiation, whether natural or artificial.

The compounds of the invention can be also used prophylactically in the situations of the risk of the exposure to the ionizing radiation.

Said exposure to the ionizing radiation includes both routine (intentional)/expected and non-intentional/non-expected exposure.

In one embodiment the invention is used in the case of acute high-dose internal or external ionizing radiation exposure.

Acute high-dose exposure can be a result of an accident, such as for example an industrial accident, or an intentional act, such as nuclear weapon detonation, dirty bomb explosion, terrorist attack involving radioactive sources, and any other criminal act with the use of ionizing radiation or radioactive material. The exposure will be external when the radiation source acts from the outside of the body, as it is for example in the case of external radiotherapy (teletherapy). The exposure will be internal if the radioactive material is ingested or inhaled by a subject as it is for example in the case of internal radiotherapy (brachytherapy) or radiation poisoning.

In another embodiment of the invention said exposure is a chronic low-dose internal or external ionizing radiation exposure.

In another embodiment of the invention said exposure is during the radiotherapy, such as radiotherapy of a cancer or tumor.

One particular variant of this embodiment said exposure is during the radiotherapy, such as radiotherapy of a cancer or tumor, and said subject is a patient undergoing radiotherapy.

The additional advantageous effect can be seen in this variant of the invention. Besides the effect of radioprotection of normal, healthy cells of said patient, the compounds of formula (I) exert also the effect of radiosensitization of abnormal cells, like tumor or cancer cells, in tissues subjected to the radiotherapy. Therefore in such variant of the invention the compounds of formula (I) provide both the radioprotecting and radiosensitizing effect. Due to this combined effect of the compounds of formula (I) the efficacy of radiotherapy can be increased or the same effect of the therapy at lower doses of the radiation can be achieved with lesser detrimental effects on healthy tissues.

Accordingly, there is also provided a quaternary pyridinium salt of formula (I) 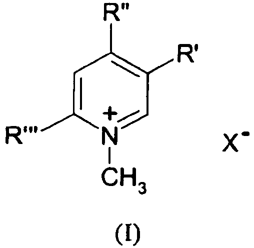 wherein R' is H, R" is H or CH₃, R"' is H or CH₃, and X⁻ is a pharmaceutically acceptable counterion, for use in the treatment of a cancer or tumor, said treatment comprising administration to a patient in need of such therapy said quaternary pyridinium salt, in an amount sufficient to inhibit radiation-induced damage, and subjecting said patient to a radiotherapy.

There is also provided a quaternary pyridinium salt of formula (I) 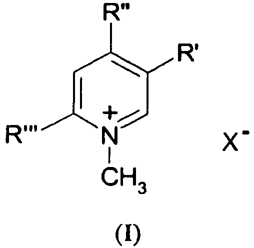 wherein R' is H, R" is H or CH₃, R''' is H or CH₃, and X⁻ is a pharmaceutically acceptable counterion, for use in a radiotherapy treatment of a living subject in a need of such treatment, said treatment comprising administration to said subject prior, during or following the radiotherapy said quaternary pyridinium salt, in an amount sufficient to inhibit radiation-induced damage.

it is also disclosed that the compounds of formula (I) will possess the ability to reduce hypoxia in tumor cells. Hypoxia in tumor cells makes them more resistant to radiation therapy, and as a consequence substantially higher radiation dose is required to kill them. One of the proposed methods of overcoming this problem in the prior art was the use of oxygen/carbon dioxide breathing in combination with nicotinamide. However, very high dose of nicotinamide required (up to 6 g) and hepato- and gastrointestinal toxicity, as well as acute toxicity, i.e. mucosal and skin reactions, have been observed.

Contrary to other compounds used as radiosensitizers, compounds of formula (I) are active as radioprotectant at relatively low doses, are easy to administer to patients, provide high increase in tissue radiosensitization, while exhibiting little systemic toxicity and having low potential for causing skin/mucosa reactions. They are also freely accessible to all hypoxic cells within the tissue subjected to radiotherapy, such as tumor cells.

The term "a cell, tissue or organ damage" as used herein refers to any harmful effect that the ionizing radiation can exert on the living body. These effects include any acute effects and long-term effects of radiation poisoning, in particular organ dysfunction or failure, multiple organ failure and death. The term "radiation damage", whenever used herein, is meant to have the same meaning as the cell or tissue damage.

The term "prevention" means protection of a living subject from any harmful effect of the ionizing radiation, including the prevention of the radiation-induced death. The term includes also the protection against damage of normal cells and long-term consequences of such damage in a patient undergoing any type of radiotherapy, as well as a medical staff member involved in such radiotherapy.

The term "treatment" as used herein includes alleviation and/or relief or controlling the symptoms, reduction of severity of symptoms and restoring the health of the subject.

The invention can be especially advantageously used for the treatment with the view of prevention of ionizing radiation-induced lethality as an acute or long-term outcome of the ionizing radiation caused damage.

The term "living subject" which is protected and/or treated from ionizing radiation damage by the method of the invention is to be understood to include living organisms which were exposed in the past, are exposed or are expected to be exposed in the future to the action of the ionizing radiation. The term "subject" includes any human or animal such as a domestic or wild animal, particularly an animal of economic importance, such as a farm animal or a livestock. In a preferred embodiment, the term "living subject" refers to a human.

The term "living subject" includes, inter alia and without any limitations, the staff of industrial plants using ionizing radiation and/or radiation sources, people traveling and/or working in the outer space, medical professionals working in the field of radiodiagnostics and/or radiotherapy, patients undergoing radiotherapy, like radiotherapy of a cancer, inhabitants of the territories radioactively contaminated due to a nuclear attack or terroristic attack, the staff of rescue or emergency services, military staff, etc.

The term "ionizing radiation" as used herein refers to particles or waves having sufficiently high energy, typically the level of few eV being sufficient, to ionize an atom or a molecule in a living body. Waves radiation includes photon radiation, such as γ rays. Particle radiation includes the radiation of energy emitted in a form of electrically charged particles, such as α and β particles and protons. Particle radiation includes the radiation of energy emitted in a form of neutrons and neutrinos. Such particle radiation may be emitted by radioactive material, i.e. the material capable of emitting radioactive particles as a result of radioactive decay.

The radiation damage caused by ionizing radiation may result from the exposure to a radiation source, such as internal or external exposure.

Internal exposure may be caused by ingestion or inhalation of a material emitting ionizing radiation (radiation source), such as radioactive material. Such ingestion or inhalation can be accidental or intentional. Accidental ingestion or inhalation is also called a radiation-poisoning. Internal exposure may be caused as well by insertion or implantation of an radioactive material into a body, such as in the course of medical therapy. Such internal exposure may be chronic (long-term) or acute.

The term "external exposure to ionizing radiation" is meant to encompass the radiation that may be caused by natural or artificial (human-made) radiation sources. Natural ionizing radiation includes natural background radiation, like solar, cosmic or external terrestrial radiation. External terrestrial radiation includes the radiation emitted by naturally present radionuclides, as well as the human-made radiation. External terrestrial radiation includes the background radiation of the radon gas, which is emitted by the decay of radioactive radium and is present in the living environment.

Human-made radiation includes that emitted by industrial radioactive wastes, radioactively contaminated working or living environment, radiation sources and radioactive materials used in medicine and industry for diagnostic and measurement purposes, radiation sources used in medicine for therapeutic purposes.

In the field of medicine, especially encompassed are radiation sources used for eradication of cancers or tumors, and radiation sources used in nuclear medicine for diagnostic or therapeutic indications. In the case of diagnostic or therapeutic uses in medicine, whether accidental or intentional, the term "living subject" includes both patients and medical personnel.

Exposure to external ionizing radiation includes also any routine exposure to radiation in industrial plants, such as for example nuclear power stations or radioactive raw-materials mines.

Exposure to external ionizing radiation includes further any exposure during the outer space (cosmic) flights and expeditions.

Exposure to external ionizing radiation includes further any exposure to the radiation being a result of generally any hostile attack or the danger thereof with the use of nuclear weapon explosions and/or radioactive material poisoning, in particular a terrorist nuclear attack or the war attack resulting in the contamination of a living environment. In the case of a hostile attack the term "living subject" includes both civil targets/victims of the attack and the defense/rescue staff involved.

The term "radiotherapy", also called "radiation therapy", as used herein, is defined as any therapeutic use of ionizing high-energy from gamma rays, electrons, protons, neutrons, and other sources to kill cancer cells and shrink tumors. Radiation may come from a machine located outside the body (external-beam radiation therapy), or it may come from radioactive material placed in the body near cancer or tumor (internal radiation therapy, implant radiation, or brachytherapy).

External radiation therapy (radiation therapy using sources from outside the body), also called external-beam radiation, is a radiation therapy that uses a machine to aim high-energy rays at the cancer or tumor.

Internal radiation therapy (radiation therapy using radioactive materials placed inside the body) is a procedure in which radioactive material sealed in needles, seeds, wires, or catheters is placed directly into or near cancer or tumor.

The use of the compounds of the above formula (I) in any of the above ionizing radiation therapy procedures is contemplated and encompassed by the present invention.

In one aspect of the invention, the compound of formula (I) is administered alone.

Generally, the compound of formula (I) can be also administered in combination with any other radioprotectants, including free-radical scavengers, such as for example amifostine and other aminothiole radioprotectants, vitamin E, vitamin C, selenium, melatonin, 5-androstenediol, curcumin, alpha-phenyl-tert-butylnitrone, a flavonoid, or a nitroxide.

The compound of formula (I) can be also administered in combination with any agent capable of removing radioactive substances from the body, such as radiogardase (prussian blue), pentetate calcium trisodium (Ca-DTPA) and pentetate zinc trisodium (Zn-DTPA).

The compound of formula (I) can be also administered in combination with any agent capable of inhibiting apoptosis, or growth factors, such as G-CSF and GM-CSF (filgrastim).

Particular compounds of formula (I) useful in the present invention are selected from 1,2-dimethylpyridinium, 1,4-dimethylpyridinium, and 1,2,4-trimethylpyridinium salts.

Preferred salts include chloride, benzoate, salicylate, acetate, citrate and lactate. Especially preferred are chloride salts, due to physiological acceptability of the chloride anion.

Some of the compounds of formula (I) are commercially available, for example 1-methylnicotinamide chloride (from Sigma). Alternatively, the compounds can be readily prepared from commercially available compounds (including nicotinamide and nicotinic acid) by synthetic methods well-known to the person skilled in the art. Such methods would include synthesis from appropriately substituted pyridine compounds without methyl group at the pyridine nitrogen atom, i.e. in the position I of the pyridine ring. Quaternary pyridinium compounds of formula (I) wherein X⁻ represents halogen anion can be prepared starting from corresponding pyridine compounds by direct methylation with methyl halogenide in a manner known *per se*. Quaternary pyridinium compounds of formula (I) wherein X⁻ represents chloride anion can be prepared by direct methylation with methyl chloride, such as disclosed in AT131118, GB348345, US3614408, and US4115390. Quaternary pyridinium compounds of formula (I) wherein X⁻ is a non-halogen anion can be prepared by substitution of a halogen anion to another anion, for example by treatment with a salt of such another anion, such as for example sodium or silver salt of another anion. As an illustration, lactates and acetates of the compounds of formula (I) can be prepared by the treatment of a halogenide, preferably chloride, with silver lactate or acetate, respectively. Salicylates of the compounds of formula (I) can be prepared by the treatment of a halogenide, preferably chloride, with sodium salicylate.

In accordance with the invention, the compounds of formula (I) can be administered orally or by injection, using any pharmaceutical dosage forms known for the person skilled in the art for such administration.

Preferred manner of administration is oral administration.

For oral administration both solid and liquid formulations can be used. Solid formulations include conventional tablets, capsules, troches, powders or granulates for direct ingestion or for reconstitution in liquids, such as water or juices. Any suitable conventional excipients can be used for the preparation of such solid forms. Liquid forms for oral administration include in particular aqueous solutions, with the addition of any conventional excipients, such as for example flavours and/or sweeteners.

Another preferred manner of administration is parenteral administration, especially by continuous infusion or a single bolus.

The administration by injection includes bolus intravenous injection, continuous intravenous infusion, as well as subcutaneous injection. Any suitable injections formulations can be used, such as for example aqueous saline solutions, buffered saline solutions, etc. using conventional excipients known from the art, such as preservatives, isotonic agents, buffers, etc.

The administration, in a single dose or in multiple doses can be prior or after each radiation episode and can be continued in cycles, such as everyday or every several days. The administration can be also continued after the radiation episode for several days or weeks. In the case of long-term radiation exposure the administration can be during the whole exposure period and can be continued after cessation of the exposure for several days or weeks.

Actual dosage levels of the compound of formula (I) in accordance with this invention may be varied so as to obtain an amount of the active ingredient which is effective to achieve the desired therapeutic response for a particular patient, composition, and mode of administration, without being toxic to the patient.

Dosage regimen in the case of the administration as a protective measure in the case of the routine, expected or predictable long-term exposure to the low-dose ionizing radiation can be in the range of about 0,01 to 10 g of the compound of formula (I) per day. The compound can be administered as a protective measure before the onset to the exposure in one single dose, or alternatively in divided doses, during the period of such exposure and optionally for some period after cessation of the exposure. The administration can be preferably repeated every day.

In the case of expected acute exposure, such as for example the exposure of a professional rescue or emergency staff after accidents involving ionizing radiation, the compound should be administered prior the expected exposure, in one single dose or in multiple doses and preferably its administration should be continued for the whole period of exposure. In the case of accidental acute exposure, the compound should be administered to the victim of such accidental exposure as quickly as possible after the exposure had taken place and conveniently should be continued daily for some period, such as several days or weeks after the exposure. In such cases a dosage regimen of about 0,02 to 20 g per day is contemplated, depending on the radiation dose absorbed by the organism.

Dosage regimens in the case of administration as a part of a radiotherapy procedure can be in the range of about 0,02 to 20 g of the compound of formula (I) in a one single dose before irradiation, such as 1 hour, 30 minutes or 15 minutes before irradiation. In particular, dosages such as 5 mg to 20 g can be used, or 5 mg to 10 g, In some embodiments, a dose the compound of formula (I) is about 1 g, or about 2 g, or about 3 g, or about 4 g, or about 5g, or about 6 g, or about 7 g, or about 8 g, or about 9 g, or about 10 g, or about 11 g, or about 12 g, or about 12 g, or about 13 g, or about 14 g, or about 15 g, or about 16 g, or about 17 g, or about 18 g, or about 19 g, or about 20 g.

Generally, the dose of the compound of the formula (I) shall be dependent on the absorbed or expected dose of the radiation. The selected dosage level will also depend upon a variety of factors including the activity of the particular compound of the present invention employed, the time of administration, the rate of excretion of the particular compound being employed, the duration of the treatment, other drugs, compounds or materials used in combination with the particular compound, the age, sex, weight, condition, general health and prior medical history of the patient being treated, and like factors well known in the medical arts.

A medical doctor, e.g., physician or veterinarian, having ordinary skill in the art can readily determine and prescribe the effective amount of the pharmaceutical composition required.

The following examples are included to exemplify and illustrate the invention and its preferred embodiments.

### Examples

The survival rate of irradiated mice treated with the compound in comparison with control was used as a measure of a radioprotective effect.

### Example 1

The effect of the administration of 1,4-dimethylpyridinium chloride (DMP)

The methodology followed was as described in B Hou. Z.W. Xu, C.W. Yang, Y. Gao, S.F. Zhao, C.G. Zhang: Protective effects of inosine on mice subjected to lethal total-body ionizing irradiation. J Radiat Res 48, 57-62, 2007. In brief, six-to-eight-week old male BALB/c mice obtained from the Nofer Institute of Occupational Medicine, Lode, Poland were used for the experiments. Whole bodies of the animals were exposed to a single irradiation (at the Military Institute of Chemistry and Radiometry) with gamma rays from the ⁶⁰Co source at 7.2 Gy/h mean dose rate to obtain the absorbed dose of 7.5 Gy per mouse. The absorbed doses were verified using thermo luminescent dosimeters implanted subcutaneously in the middle abdominal region. All the mice were maintained under specific pathogen-free conditions. Survival of the mice was assessed during 30 days following the irradiation.

The mice were divided into irradiated control group receiving no treatment, and irradiated treatment group receiving DMP. The treatment group was divided into two subgroups, the first group in which the animals received the tested compound starting from day 7 before the irradiation, and the second group in which the animals received the tested compound starting from day 7 after the irradiation. The compound was given dissolved in drinking water at 100 mg/kg of body weight/24 h until day 30 post irradiation.

The results of the tests are presented on the Fig.1 of the drawing. It can be seen that the compound tested exerts its protective activity both when administered prior irradiation and when administered after irradiation.

## Claims

1. A quaternary pyridinium salt of formula (I) 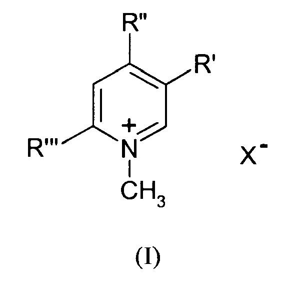 wherein R' is H, R" is H or CH₃, R''' is H or CH₃, and X⁻ is a pharmaceutically acceptable counterion, for use in the treatment or prevention of a cell, tissue or organ injury in a living subject due to the exposure to ionizing radiation.

2. The compound of formula (I) for use according to claim 1 wherein said exposure is an acute high-dose internal or external radiation exposure.

3. The compound of formula (I) for use according to claim 1 wherein said exposure is a chronic internal or external radiation exposure.

4. The compound of formula (I) for use according to claims 1 to 3 wherein said exposure is the exposure of a patient during external or internal radiotherapy.

5. The compound of formula (I) for use according to claim 1 wherein said subject is a medical professional involved in radiodiagnostics and/or radiotherapy.

6. The compound of formula (I) for use according to claim 1 wherein the compound of formula (I) is selected from 1,2-dimethylpyridinium, 1,4-dimethylpyridinium, and 1,2,4-trimethylpyridinium salts.

7. The compound of formula (I) for use according to claim 1 by oral administration.

8. The compound of formula (I) for use according to claim 1 by parenteral administration.

9. The compound of formula (I) for use according to claim 1 for the prevention of radiation-induced death.

10. The compound of formula (I) for use according to claim 1 for the treatment of radiation poisoning.

11. The compound of formula (I) for use according to claim 1 for the treatment of acute radiation sickness.

12. A quaternary pyridinium salt of formula (I) 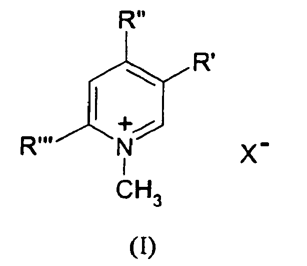 wherein R' is H, R" is H or CH₃, R''' is H or CH₃, and X⁻ is a pharmaceutically acceptable counterion for use in the treatment of a cancer or tumor, said treatment comprising administration to a patient in need of such therapy said quaternary pyridinium salt, in an amount sufficient to inhibit radiation-induced damage, and subjecting said patient to a radiotherapy procedure.

13. A qaternary pyridinium salt of formula (I) 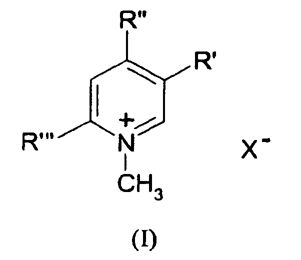 wherein R' is H, R" is H or CH₃, R''' is H or CH₃, and X⁻ is a pharmaceutically acceptable counterion for use in a radiotherapy treatment of a living subject in a need of such treatment, said treatment comprising administration to said subject prior, during or following the radiotherapy said quaternary pyridinium salt, in an amount sufficient to inhibit radiation-induced damage.

## Patentansprüche

1. Ein quaternäres Pyridiniumsalz der Formel (I) 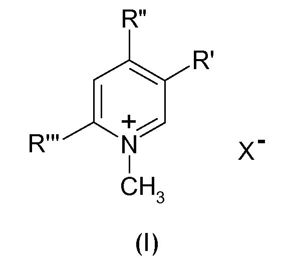 worin R' für H steht, R" für H oder CH₃ steht, R'" für H oder CH₃ steht, und X⁻ für ein pharmazeutisch annehmbares Gegenion steht, zur Verwendung in der Behandlung oder Prophylaxe von einer Zell-, Gewebe- oder Organschädigung in einem lebenden Subjekt die durch die Exposition gegenüber ionisierender Strahlung verursacht ist.

2. Verbindung der Formel (I) zur Verwendung nach Anspruch 1, wobei gesagte Exposition eine akute Hochdosis-Exposition gegenüber innere oder äußere Strahlung ist.

3. Verbindung der Formel (I) zur Verwendung nach Anspruch 1, wobei gesagte Exposition eine chronische Exposition gegenüber innere oder äußere Strahlung ist.

4. Verbindung der Formel (I) zur Verwendung nach Ansprüche 1 bis 3, wobei gesagte Exposition eine Exposition eines Patienten während der äußeren oder inneren Strahlentherapie ist.

5. Verbindung der Formel (I) zur Verwendung nach Anspruch 1, wobei gesagte Subjekt ist ein medizinisches Fachmann der in der Röntgendiagnostik und/oder Strahlentherapie beteiligt ist.

6. Verbindung der Formel (I) zur Verwendung nach Anspruch 1, wobei gesagte Verbindung der Formel (I) aus 1,2-Dimethylpyridinium, 1,4-Dimethylpyridinium und 1,2,4-Trimethylpyridinium Salze ausgewählt ist.

7. Verbindung der Formel (I) zur Verwendung nach Anspruch 1 durch orale Verabreichung.

8. Verbindung der Formel (I) zur Verwendung nach Anspruch 1 durch parenterale Verabreichung.

9. Verbindung der Formel (I) zur Verwendung nach Anspruch 1 zur Prophylaxe von durch Strahlung induzierten Tod.

10. Verbindung der Formel (I) zur Verwendung nach Anspruch 1 zur Behandlung von Strahlenkrankheit.

11. Verbindung der Formel (I) zur Verwendung nach Anspruch 1 zur Behandlung der akuten Strahlenkrankheit.

12. Ein quaternäres Pyridiniumsalz der Formel (I) 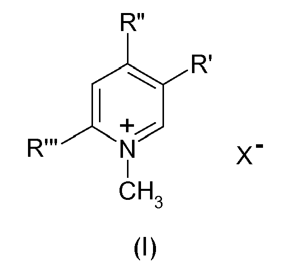 worin R' für H steht, R" für H oder CH₃ steht, R"' für H oder CH₃ steht, und X⁻ für ein pharmazeutisch annehmbares Gegenion steht, zur Verwendung in der Behandlung eines Krebs oder Tumor, gesagte Behandlung umfassend Verabreichung an einen Patienten der eine solche Therapie braucht, gesagtes quaternären Pyridiniumsalz in einer Menge die ausreicht, um strahlungsbedingten Schäden zu verhindern, und Unterwerfung von Patienten zu einer Strahlentherapie Verfahren.

13. Ein quaternäres Pyridiniumsalz der Formel (I) 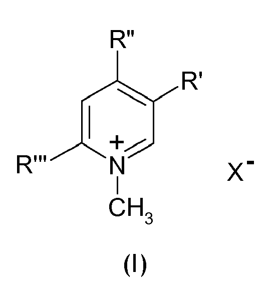 worin R' für H steht, R" für H oder CH₃ steht, R"' für H oder CH₃ steht, und X⁻ für ein pharmazeutisch annehmbares Gegenion steht, zur Verwendung in der Behandlung durch Strahlentherapie von lebenden Subjekt, der eine solche Therapie braucht, gesagte Behandlung umfassend eine Verabreichung an einen Patienten vor, während oder nach der Strahlentherapie gesagtes quaternären Pyridiniumsalz in einer Menge die ausreicht, um strahlungsbedingten Schäden zu verhindern.

## Revendications

1. Un sel de pyridinium quaternaire de la formule (I) 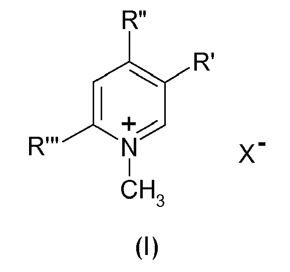 dans laquelle R' est H, R" est H ou CH₃, R"' est H ou CH₃, et X⁻ est un contre-ion pharmaceutiquement acceptable, pour utilisation dans le traitement ou la prévention d'une lésion d'une cellule, d'un tissu ou d'un organe chez un sujet vivant résultant de l'exposition à un rayonnement ionisant.

2. Le compose de la formule (I) pour utilisation selon la revendication 1, ladite exposition étant une exposition aiguë interne ou externe à forte dose de rayonnement.

3. Le compose de la formule (I) pour utilisation selon la revendication 1, ladite exposition étant une exposition chronique interne ou externe à rayonnement.

4. Le compose de la formule (I) pour utilisation selon la revendication 1, ladite exposition étant une exposition d'un patient lors d'une radiothérapie externe ou interne.

5. Le compose de la formule (I) pour utilisation selon la revendication 1, ledit sujet étant un professionnel de la santé impliqué dans radiodiagnostic et/ou radiothérapie.

6. Le compose de la formule (I) pour utilisation selon la revendication 1, le composé de formule (I) étant choisi parmi les sels de 1,2-dimethylpyridinium, 1,4-dimethylpyridinium, et 1,2,4-trimethylpyridinium.

7. Le compose de la formule (I) pour utilisation selon la revendication 1, par administration par la voie orale.

8. Le compose de la formule (I) pour utilisation selon la revendication 1, par administration par la voie parentérale.

9. Le compose de la formule (I) pour utilisation selon la revendication 1 dans la prévention de la mort induite par le rayonnement.

10. Le compose de la formule (I) pour utilisation selon la revendication 1 dans le traitement de l'empoisonnement rayonnement.

11. Le compose de la formule (I) pour utilisation selon la revendication 1 dans le traitement de de maladies dues aux radiations aiguë.

12. Un sel de pyridinium quaternaire de la formule (I) 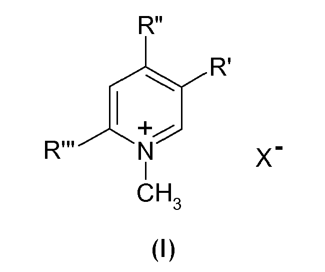 dans laquelle R' est H, R" est H ou CH₃, R"' est H ou CH₃, et X⁻ est un contre-ion pharmaceutiquement acceptable, pour utilisation dans le traitement d'un cancer ou une tumeur, ledit traitement comprenant l'administration à un patient ayant besoin d'un tel traitement ledit sel pyridinium quaternaire en une quantité suffisante pour inhiber l'endommagement par rayonnement, et soumettant ledit patient à une procédure de radiothérapie.

13. Un sel de pyridinium quaternaire de la formule (I) 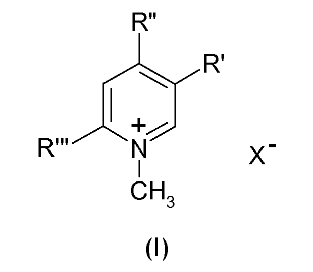 dans laquelle R' est H, R" est H ou CH₃, R"' est H ou CH₃, et X⁻ est un contre-ion pharmaceutiquement acceptable pour utilisation dans le traitement par radiothérapie d'un sujet vivant ayant besoin d'un tel traitement, ledit traitement comprenant l'administration audit sujet avant, pendant ou après la radiothérapie ledit sel pyridinium quaternaire en une quantité suffisante pour inhiber l'endommagement par rayonnement.
